# EUROPEAN PATENT APPLICATION

(11) **EP 4 252 814 A1**
(43) Date of publication of application: **04.10.2023**
(21) Application number: 21913619.9
(22) Date of filing: 24.11.2021
(51) Int. Cl.: A61M 25/00

(54) **CATHETER AND METHOD FOR PREPARING CATHETER TRANSITION STRUCTURE**

(30) Priority: 31.12.2020 CN 202011619560
(71) Applicant: MicroPort NeuroTech (Shanghai) Co., Ltd., Shanghai 201318 (CN)
(72) Inventor: LIN, Heng, Shanghai 201318 (CN); LIU, Yunyun, Shanghai 201318 (CN); LIU, Qinglong, Shanghai 201318 (CN); LUO, Xueli, Shanghai 201318 (CN); LIU, Yumei, Shanghai 201318 (CN); SUN, Li, Shanghai 201318 (CN)
(74) Representative: Marks & Clerk LLP
(86) International application number: PCT/CN2021/132608
(87) International publication number: WO 2022/142877

(57) **Abstract**

A catheter (100) and a method of making a transition structure (200) of a catheter. The catheter (100) includes at least one polymeric layer including at least one transition structure (200). Each transition structure (200) is a tubular structure including a homogeneous material zone (1) and a heterogeneous material zone (2). Any continuous closed region of the homogeneous material zone (1) is referred to as a first sub-region (1011) and any continuous closed region of the heterogeneous material zone (2) as a second sub-region (2001), and any axial section of the transition structure (200) includes at least one second sub-region (2001). A leading section and/or a trailing section of the transition structure (200) include(s) at least one first sub-region (1011) and at least one second sub-region (2001). The catheter has smooth transitioning of flexibility/stiffness and mechanical properties, which can enhance the catheter's delivery performance.

## Description

### TECHNICAL FIELD

The present invention relates to the field of medical devices and, in particular, to a catheter for cerebrovascular intervention.

### BACKGROUND

In minimally-invasive interventional procedures, with the assistance of an imaging system, an implantable medical device or therapeutic drug is delivered through a catheter-based delivery system deployed in a blood vessel to a lesion site for effecting mechanical or chemical treatment thereof while causing minimal trauma. As an important component of such delivery system, catheters have been widely used in various minimally-invasive interventional procedures.

In current interventional procedures, a catheter is usually introduced through an incision made in a small blood vessel (e.g., the femoral artery or the radial artery) and advanced through the blood vessel to a target lesion site with the aid of a sheath, a guidewire or the like. In practical clinical applications, due to the tortuous nature of blood vessels, it would take some time for a surgeon to create such an access system. If a catheter can successfully pass through a particularly tortuous blood vessel site, such as a type III aortic arch or the ophthalmic artery in the head, the surgical time required can be greatly reduced.

A catheter is typically a triple-layered structure consisting of an inner layer, a reinforcing layer and an outer layer. The inner and outer layers are often fabricated from polymeric tubes, and the reinforcing layer is usually made of metallic wire or polymeric wire and embedded between the inner and outer layers. Structurally, the flexibility of such a catheter depends on the three factors: stiffness of the inner layer; strength and coverage density of the wires in the reinforcing layer; and stiffness of the outer layer. In practical clinical applications, in order to enable a catheter to be successively delivered to a target diseased site, different portions of the catheter have different stiffness, overall, it is stiffer proximally and more flexible distally, and the stiffness gradually decreases from the proximal end to the distal end, and the degrees of stiffness at different portions of the catheter is often designed according to the anatomy of blood vessels. This stiffness profile is achievable through differentiating various catheter sections through fabricating them with polymeric materials with different stiffness properties or metallic wires with different strength properties or arranged at different pitches. Polymeric materials that are commonly used to fabricate main sections of catheters include polytrafluoroethylene, polyurethane, polyamide, polyolefin, etc. A distal end portion of a catheter is usually made of a flexible polymeric material (e.g., pebax25D), and a proximal end portion of a catheter is often made of a stiff polymeric material (e.g., Pebax 72D or nylon). Structurally, if adjacent catheter sections exhibit abruptly different degrees of stiffness, compliance of the catheter will be degraded, making the catheter difficult to advance through a tortuous blood vessel and creating a risk of causing damage to the blood vessel.

In order to achieve a good delivery performance, the catheter portions of polymeric materials with different stiffness are joined together so as to enable the catheter satisfies the mechanical properties at different vessel locations. Conventional method of making catheters lacks smooth transitioning of mechanical properties between different portions, thereby affecting the delivery and safety performance.

### SUMMARY OF THE INVENTION

It is an object of the present invention to provide a catheter and a method of making a transition structure of a catheter, which overcome the problem associated with conventional fabricated catheters of unsatisfactory delivery and safety performance due to lacking of smooth transitioning of mechanical properties between different catheter sections.

To this end, the present invention provides a catheter comprising
at least one polymeric layer, wherein the polymeric layer comprises at least one transition structure that is a tubular structure, wherein the transition structure comprises a homogeneous material zone and a heterogeneous material zone, wherein: any continuous closed region of the homogeneous material zone is referred to as a first sub-region; any continuous closed region of the heterogeneous material zone as a second sub-region; and any axial section of the transition structure comprises at least one second sub-region, and wherein a leading section and/or a trailing section of the transition structure comprise(s) at least one first sub-region and at least one second sub-region.

Optionally, any axial section of the transition structure may comprise at least one first sub-region and at least one second sub-region.

Optionally, from the leading location and/or the trailing location of the transition structure at respective end(s) to an intermediate section of the transition structure, an outer surface area ratio of homogeneous material zone to heterogeneous material zone gradually decreases.

Optionally, from a first end to a second end of the transition structure, an outer surface area ratio of homogeneous material zone to heterogeneous material zone gradually decreases to a minimum value and then gradually increases.

Optionally, at least one end face of the heterogeneous material zone may define a plane which is inclined with respect to an axis of the catheter at an angle.

Optionally, the plane may be inclined with respect to the axis of the catheter at an angle ranging from 5° to 60°.

Optionally, at least one edge of the heterogeneous material zone may have a polylineal structure.

Optionally, the heterogeneous material zone may have a constant axial length in the range of 1-15 mm.

Optionally, the heterogeneous material zone may have a varying axial length with a maximum value of 3-20 mm and a minimum value of 0.5-15 mm.

Optionally, the homogeneous material zone may comprise a first polymeric region and a second polymeric region situated on opposite sides of the heterogeneous material zone, wherein the first polymeric region is made of a first polymeric material, wherein the second polymeric region is made of a second polymeric material, and wherein a stiffness of the first polymeric material is higher than a stiffness of the second polymeric material.

Optionally, stiffness of the heterogeneous material zone may be lower than stiffness of the first polymeric region and higher than stiffness of the second polymeric region.

Optionally, at least one end face of the heterogeneous material zone may define a plane which is inclined with respect to an axis of the catheter at an angle, wherein a greater stiffness difference between the first and second polymeric materials is, a smaller angle is formed.

Optionally, the heterogeneous material zone may be made of the first and second polymeric materials, which are blended and fused.

Optionally, the heterogeneous material zone may be made of the first and second polymeric materials, which are piled and joined.

Optionally, the first polymeric material may be any one of polytrafluoroethylene, polyolefin, polyurethane, poly(ether-block-amide) and polyamide, and the second polymeric material may be any one of polytrafluoroethylene, polyolefin, polyurethane, poly(ether-block-amide) and polyamide.

Optionally, the catheter may further include a reinforcing layer.

Optionally, the catheter may be a triple-layered structure comprising an inner layer, an intermediate reinforcing layer and an outer layer, wherein the inner and outer layers are polymeric layers, and wherein the inner layer comprises at least one transition structure and/or the outer layer comprises at least one transition structure.

Optionally, the inner layer may comprise at least one transition structure.

Optionally, a distal section of the inner layer may comprise the at least one transition structure.

Optionally, the outer layer may comprise at least one transition structure.

The present invention also provides a method of making a transition structure of a catheter, which comprises: cutting first and second polymeric tubes at their respective ends so that an end portion of the first polymeric tube and/or an end portion of the second polymeric tube have/has a circumferential extent which is smaller than a complete circumference, the first polymeric tube made of a first polymeric material, the second polymeric tube made of a second polymeric material, a stiffness of the first polymeric material is higher than a stiffness of the second polymeric material; and the end of the first polymeric tube and the end of the second polymeric tube are at least partially piled and joined and then subjected to a heat shrinkage process.

Optionally, the heat shrinkage process may be carried out at a temperature higher than melting points of the first and second polymeric materials.

Optionally, the heat shrinkage process may be carried out at a temperature lying between the melting point of the first polymeric material and the melting point of the second polymeric material.

Optionally, the method may further comprise, prior to the piling and joining, stretching the end of the first polymeric tube and/or the end of the second polymeric tube, so that for each tube, a wall thickness at a stretched end is smaller than a wall thickness at remaining positions of the tube.

In summary, the present invention provides a catheter comprising at least one polymeric layer. The polymeric layer comprises at least one transition structure, which is a tubular structure comprising a homogeneous material zone and a heterogeneous material zone. Any continuous closed region of the homogeneous material zone is referred to as a first sub-region and any continuous closed region of the heterogeneous material zone as a second sub-region, and any axial section of the transition structure comprises at least one second sub-region, and a leading section and/or a trailing section of the transition structure comprise(s) at least one first sub-region and at least one second sub-region. The present invention also provides a method of making such a transition structure. The catheter and the method offer at least the advantages as follows:
1. In the catheter, there is smooth transitioning of flexibility/stiffness and mechanical properties around the interface between the polymeric materials, which enables the catheter to have good overall mechanical properties and enhanced delivery and safety performance.
2. The catheter can satisfy mechanical property requirements of various blood vessel sites and is avoided from easy kinking at the location where mechanical properties change.
3. Stress concentration will not easily occur even when two materials exhibiting a significant stiffness difference are joined together, allowing reduced numbers of different polymeric materials used and joined sections in the catheter.
4. The end of the polymeric tubes may be stretched, so that a wall thickness at a stretched end is smaller than a wall thickness at remaining positions of the tub. This enables thickness control at the joint and avoids the catheter from having an excessive outer diameter.
5. The presence of the heterogeneous material zone enables higher strength at the joint, resulting in improved safety performance of the catheter.

### BRIEF DESCRIPTION OF THE DRAWINGS

Fig. 1 is a schematic illustration of a catheter according to a preferred embodiment of the present invention.
Fig. 2 shows an axial projection of a transition structure in a catheter according to a preferred embodiment of the present invention;
Fig. 3 is a cross-sectional view of a section of a catheter according to a preferred embodiment of the present invention.
Fig. 4 is an unrolled view of a transition structure in a catheter according to a preferred embodiment of the present invention.
Fig. 5 is an unrolled view of a transition structure in a catheter according to a preferred embodiment of the present invention.
Fig. 6 is an unrolled view of a transition structure in a catheter according to a preferred embodiment of the present invention.
Fig. 7 shows an axial projection of a transition structure in a catheter according to a preferred embodiment of the present invention.
Fig. 8 is an unrolled view of a transition structure in a catheter according to a preferred embodiment of the present invention.
Fig. 9 is a cross-sectional view of a transition structure in a catheter according to a preferred embodiment of the present invention.

In these figures,
100, a catheter; 200, a transition structure; 201, a leading location; 202, a trailing location; 210, a leading section; 220, a trailing section; 1, a homogeneous material zone; 2, a heterogeneous material zone; 101, a first polymeric region; 102, a second polymeric region; 1011 and 1021, first sub-regions; 2001, a second sub-region; 3, a proximal tube; 4, a distal tube; 1001, an inner layer; 1002, a reinforcing layer; 1003, an outer layer; and 1004, a lumen.

### DETAILED DESCRIPTION

Objects, advantages and features of the present invention will become more apparent upon reading the following more detailed description of the present invention, which is made with reference to the accompanying drawings. Note that the figures are provided in a very simplified form not necessarily drawn to exact scale and for the only purpose of facilitating easy and clear description of the embodiments.

As used herein, the singular forms "a", "an" and "the" include plural referents, and the term "plurality" is employed in the sense of "two or more", unless the context clearly dictates otherwise. As used herein, the term "or" is generally employed in the sense of "and/or", unless the context clearly dictates otherwise. The term "proximal end" generally refers to an end closer to an operator, and the term "distal end" generally refers to an end closer to a lesion in a patient, unless the context clearly dictates otherwise. Those of ordinary skill in the art can understand the specific meanings of the above-mentioned terms herein, depending on their context. Throughout the several views, like numerals indicate like elements.

### Embodiment 1

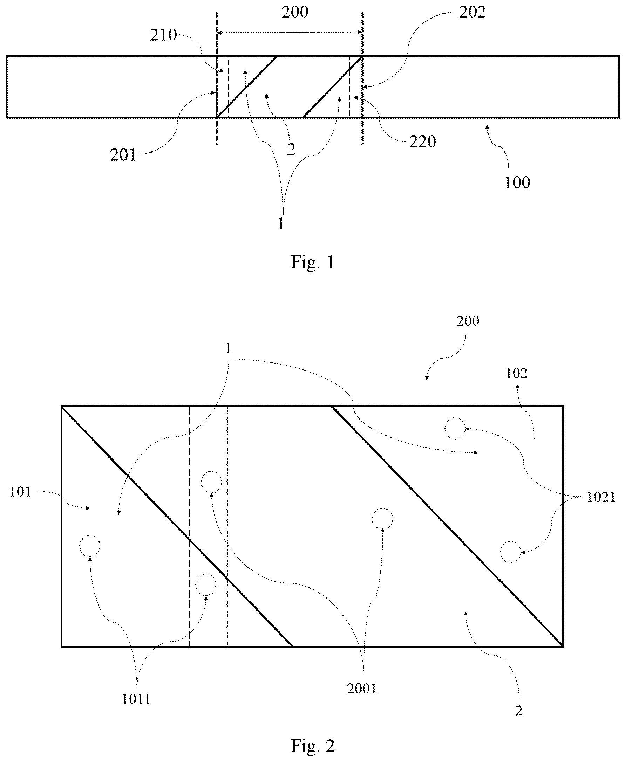

In Embodiment 1, there is provided a catheter 100 including at least one polymeric layer comprising at least one transition structure 200. Fig. 1 is a schematic illustration of the catheter 100 according to the Embodiment 1 As shown in Fig. 1, the transition structure 200 is a tubular structure extending from a leading location 201 to a trailing location 202. In the transition structure 200, the portion between the leading location 201 and the trailing location 202 is referred to as an intermediate section. The transition structure 200 includes a homogeneous material zone 1 and a heterogeneous material zone 2.

Fig. 2 shows an axial projection of the transition structure 200 in the catheter 100 on a certain plane. In the projection shown in Fig. 2, the transition structure 200 is symmetrical in the front-rear direction, however, in other embodiments, the transition structure 200 may not have such a projection that is symmetrical in the front-rear direction. As shown in Fig. 2, the homogeneous material zone 1 further includes a first polymeric region 101 and a second polymeric region 102. The first polymeric region 101 is made of a first polymeric material and the second polymeric region 102 of a second polymeric material. Stiffness of the first polymeric material is higher than stiffness of the second polymeric material. In some other embodiments, the stiffness of the first polymeric material may be lower than the stiffness of the second polymeric material.

Any continuous closed region of the transition structure 200 may be referred to as a sub-region. The transition structure 200 is actually a tubular structure, and a sub-region thereof is defined as a continuous closed portion, which can be "obtained by cutting" through the tube in a direction perpendicular to its wall along a line delineating any arbitrary continuous closed shape on an outer surface of the tubular structure. Herein, any continuous closed region of the homogeneous material zone is referred to as a first sub-region 1011 (or 1021), and any continuous closed region of the heterogeneous material zone 2 as a second sub-region 2001.

In Fig. 2, the dotted circles indicate sub-regions, and the quadrilateral region delimited by the two oblique lines is the heterogeneous material zone 2. Moreover, the two triangular regions respectively indicate the first polymeric region 101 and the second polymeric region 102 that make up the homogeneous material zone 1. Any continuous closed region in the first polymeric region 101 is referred to as a first sub-region 1011, and any continuous closed region in the second polymeric region 102 as a first sub-region 1021. That is, each first sub-region 1011 is located at the first polymeric region 101, and each first sub-region 1021 is located at the second polymeric region 102. Moreover, any continuous closed region in the heterogeneous material zone 2 is referred to as a second sub-region 2001. As can be seen from Fig. 2, an arbitrarily long axial section of the transition structure 200, such as that delimited by the dashed straight lines in the figure, includes at least one second sub-region 2001. Moreover, in an axial section of the transition structure 200 extending from an axial end provided by the first polymeric region 101 (located at the leading location 201 of the transition structure 200) to an end position of the first polymeric region 101 (located in the intermediate section of the transition structure 200), an arbitrarily long axial section, such as that delimited by the dashed straight lines in Fig. 2, includes at least one first sub-region 1011 and at least one second sub-region 2001. Likewise, in an axial section of the transition structure 200 extending from an axial end provided by the second polymeric region 102 (located at the trailing location of the transition structure 200) to an end position of the second polymeric region 102 (located in the intermediate section of the transition structure 200), an arbitrarily long axial section includes at least one first sub-region 1021 and at least one second sub-region 2001. In Embodiment 1, the homogeneous material zone 1 is provided at both ends of the transition structure 200. In some other embodiments, the transition structure 200 may include only the first polymeric region 101 that may provide either end thereof. Alternatively, it may include only the second polymeric region 102 that may provide either end thereof. In Embodiment 1, the leading location 201 is located at the end of the transition structure 200 provided by the first polymeric region 101, and a leading section 210 is defined as the segment adjacent to the leading location 201. It is to be noted that the leading section 210 is a relatively short section in the proximity of the leading location 201, which extends from the leading location 201 to a chosen location located in the intermediate section of the transition structure 200 around the leading location 201. The trailing location 202 is located at the end of the transition structure 200 provided by the second polymeric region 102, and a trailing section 220 is defined as the segment adjacent to the trailing location 202. It is to be noted that the trailing section 220 is a relatively short section in the proximity of the trailing location 202, which extends from the trailing location 202 to a chosen location located in the intermediate section of the transition structure 200 around the trailing location 202. In Embodiment 1, each of the leading section 210 and the trailing section 220 includes at least one first sub-region 1011 (and/or first sub-region 1021) and at least one second sub-region 2001. In some other embodiments, the leading section 210 of the transition structure 200 may include at least one first sub-region 1011 (or first sub-region 1021) and at least one second sub-region 2001, whilst the trailing section 220 may include only at least one second sub-region 2001. Alternatively, in some other embodiments, the trailing section 220 of the transition structure 200 may include at least one first sub-region 1011 (or first sub-region 1021) and at least one second sub-region 2001, whereas the leading section 210 may include only at least one second sub-region 2001.

In Embodiment 1, the heterogeneous material zone 2 forms an oblique tubular structure. That is, both end faces of the heterogeneous material zone 2 define planes inclined at angles with respect to an axis of the catheter, the angles may be either equal or unequal and may range from 5° to 60°. In some other embodiments, only one of the planes defined by the end faces of the heterogeneous material zone 2 may be inclined with respect to the axis of the catheter, while the other plane may be perpendicular to the axis of the catheter.

The heterogeneous material zone 2 may be formed of a blend of the first and second polymeric materials. For example, fabrication of the catheter may involve forming an integral tube from two or more different materials by heat shrinkage. In the process of heat shrinkage, when heated to a temperature higher than all the melting points of the different materials, the materials will diffuse into each other through their interface(s) of contact, leading to fusion and blending of them. Alternatively, the heterogeneous material zone 2 may be a composite structure consisting of laminates of the first and second polymeric materials. For example, laminates of one polymeric material with beveled end faces may be inserted between laminates of the other polymeric material with or without beveled end faces, and the resulting lamination may be subjected to a heat shrinkage process performed at a temperature, which is not higher than a higher one of the melting points of the materials but is higher than the lower one of the melting points of the materials. As a result of such a heat shrinkage process, the materials will not be blended but will be bonded together to form a composite structure. In some embodiments, the heterogeneous material zone 2 may be formed of the first and second polymeric materials which are fused and blended. In some other embodiments, the heterogeneous material zone 2 may be a composite structure consisting of laminates of the first and second polymeric materials which are bonded together.

In Embodiment 1, stiffness of the heterogeneous material zone 2 lies between the stiffness of the first polymeric material and the stiffness of the second polymeric material. In some other embodiments, the stiffness of the heterogeneous material zone 2 may be slightly higher than the stiffness of the first polymeric material and the stiffness of the second polymeric material. The presence of the heterogeneous material zone 2 enables higher strength at joints between different sections of the catheter, resulting in improved safety performance thereof.

As shown in Fig. 2, in Embodiment 1, an outer surface area ratio of the first polymeric region 101 or second polymeric region 102 to the heterogeneous material zone 2 gradually deceases from the leading location 201 or trailing location 202 to the intermediate section of the transition structure 200, and there is an axial segment of the intermediate section of the transition structure 200 provided only by the heterogeneous material zone 2. In other embodiments, the outer surface area ratio of the first polymeric region 101 (or second polymeric region 102) to the heterogeneous material zone 2 may gradually decrease from one end to the other end of the transition structure 200. Alternatively, in some other embodiments, the outer surface area ratio of the first polymeric region 101 (or/and second polymeric region 102) to the heterogeneous material zone 2 may decrease to a minimum and then gradually increase from one end to the other end of the transition structure 200.

In the polymeric layer of the catheter 100 in Embodiment 1, the presence of the transition structure 200 enables smooth transitioning of flexibility/stiffness and mechanical properties of the catheter 100, which imparts to the catheter 100 better overall mechanical properties and enhanced delivery and safety performance.

Furthermore, in order to satisfy the mechanical property requirements of various blood vessel sites, it is necessary for the catheter 100 to have different degrees of stiffness at different portions. This requires it to be made of multiple polymeric materials with different degrees of stiffness and/or of different types, which are joined together. However, if two of the joined polymeric materials show an abrupt stiffness difference, the catheter 100 will tend to kink at the joint thereof, leading to a reduced lumen size or even permanent deformation. In order to overcome this problem, the at least one polymeric layer in the catheter 100 is generally made of multiple polymeric materials, which are joined together at multiple joints, in order to meet the mechanical property requirements of various blood vessel sites and enables smooth transition of mechanical properties. At each of the joints, the transition structure 200 as described in Embodiment 1 may be employed to avoid stress concentration even when the joined two materials have quite different degrees of stiffness. This allows reduced numbers of different polymeric materials used and joined sections in the catheter while enabling desirable smooth transitioning of mechanical properties.

### Embodiment 2

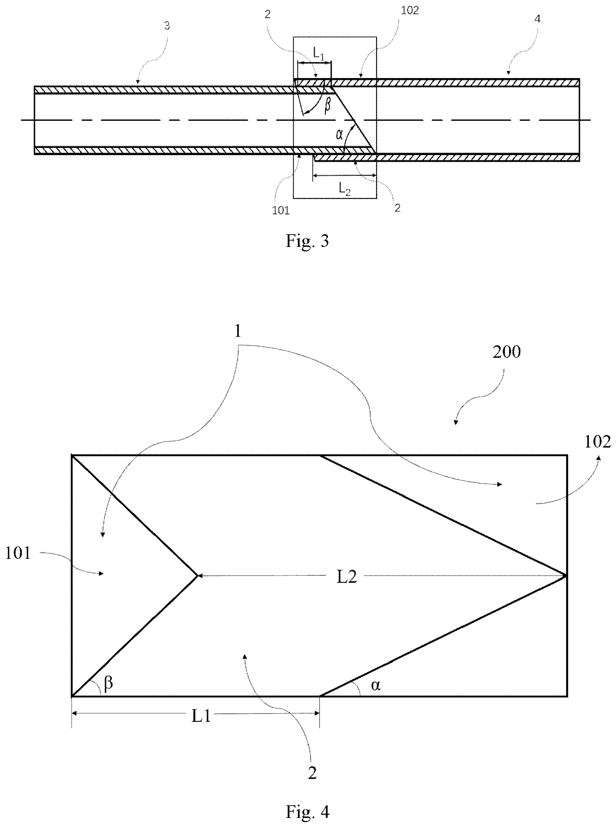

Fig. 3 shows a partial axial cross-sectional view of a catheter 100 including a transition structure 200, as indicated by the rectangular box in the figure. There is a proximal tube 3 at a proximal end of the transition structure 200 and a distal tube 4 at a distal end of the transition structure 200. The proximal tube 3 is made of a first polymeric material and the distal tube 4 of a second polymeric material. Stiffness of the first polymeric material is higher than that of the second polymeric material. A distal extension portion of the proximal tube 3 forms a first polymeric region 101, and a proximal extension portion of the distal tube 4 forms a second polymeric region 102. Portions of the proximal tube 3 and the distal tube 4, where they come into contact with each other, or where their materials are blended, form a heterogeneous material zone 2. For ease of distinction, the heterogeneous material zone 2 is shown in Fig. 3 as a composite material layer consisting of the first and second polymeric materials which are overlapped and bonded to each other, as an example. In other embodiments, in the heterogeneous material zone 2, the first and second polymeric materials may be blended together. In the transition structure 200, end faces of the heterogeneous material zone 2 facing the homogeneous material zone 1 define planes inclined with respect to an axis of the catheter. The plane defined by the end face of the heterogeneous material zone 2 facing the first polymeric region 101 is inclined with respect to the axis of the catheter at an angle β, and the plane defined by the end face of the heterogeneous material zone 2 facing the second polymeric region 102 is inclined with respect to the axis of the catheter at an angle α. The heterogeneous material zone 2 has a minimum axial length L1 and a maximum axial length L2. β may be equal to α, or not. L1 may be equal to L2, or not. When β is equal to α, L1 is equal to L2. When the catheter 100 has an inner diameter of 0.013" to 0.029", L1 and L2 are preferred to range from 1 mm to 3 mm, and α and β are preferred to range from 45° to 60°. When the inner diameter of the catheter lies between 0.055" and 0.090", L1 and L2 are preferred to range from 6 mm to 15 mm, and α and β are preferred to range from 5° to 60°. In some other embodiments, L1 and L2 may be 0.5-20 mm. In some embodiments, the heterogeneous material zone may have a constant axial length (i.e., L1=L2) in the range of 1-15 mm, such as 1 mm, 3 mm, 5 mm, 7.5 mm, 10 mm, 12 mm, 15 mm or the like. In some embodiments, the heterogeneous material zone may have a varying axial length (i.e., L1≠L2). Additionally, L1 may be in the range of 0.5-15 mm, such as 0.5 mm, 2 mm, 5 mm, 6 mm, 8 mm, 11 mm, 12.5 mm, 15 mm or the like, and L2 may be in the range of 3-20 mm, such as 3 mm, 5 mm, 7 mm, 10 mm, 12 mm, 15 mm, 17.5 mm, 20 mm or the like.

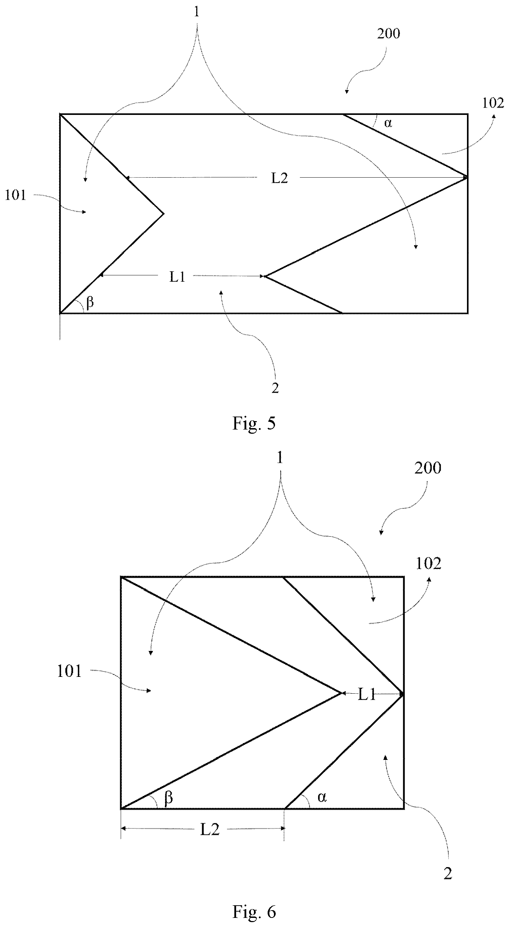

Fig. 4 shows the transition structure 200 of Fig. 3 after it is cut along an axial line and unrolled flat. From Fig. 4, the locations and relationships of the L1, L2, α and β can be more intuitively understood. In Fig. 4, the heterogeneous material zone 2 is cut along its minimum axial length. In Embodiment 2, the transition structure 200 is a symmetrical structure. As can be seen from Fig. 4, the axially cut and unrolled transition structure 200 is vertically symmetrical. As shown in Fig. 5, in some other embodiments, the transition structure 200 may be not a symmetrical structure. For example, a line connecting a trailing location of the first polymeric region 101 (the right apex of the left triangle in Fig. 5) and an end location of the second polymeric region 102 (the rightmost intersection in Fig. 5) may be not parallel to the axis of the catheter. As can be also seen from Fig. 4, an arbitrarily long axial section of the transition structure 200 must include a second sub-region 2001. In an axial portion of the transition structure 200 extending from an axial end thereof provided by the first polymeric region 101 (located at a leading location 201 of the transition structure 200) to an end of the first polymeric region 101 (located in an intermediate section of the transition structure 200), an arbitrarily long axial section includes at least one first sub-region 1011 and at least one second sub-region 2001. Likewise, in an axial portion of the transition structure 200 extending from an axial end thereof provided by the second polymeric region 102 (located at the trailing location of the transition structure 200) to an end of the second polymeric region 102 (located in the intermediate section of the transition structure 200), an arbitrarily long axial section includes at least one first sub-region 1021 and at least one second sub-region 2001. In Embodiment 2, α and β are different non-right angles. In other embodiments, α and β may be the same with each being a non-right angle. In other embodiments, β is not 90°, but α may be 90°, in this case, the transition structure 200 includes only the first polymeric region 101 and the heterogeneous material zone 2. In the axial portion of the transition structure 200 extending from the axial end provided by the first polymeric region 101 (located at the leading location 201 of the transition structure 200) to the end position of the first polymeric region 101 (located in the intermediate section of the transition structure 200), an arbitrarily long axial section includes at least one first sub-region 1011 and at least one second sub-region 2001. Likewise, when α is not 90°, β may be 90°. In this case, the transition structure 200 includes only the second polymeric region 102 and the heterogeneous material zone 2, and in the axial portion of the transition structure 200 extending from the axial end provided by the second polymeric region 102 (located at the trailing location of the transition structure 200) to the end position of the second polymeric region 102 (located in the intermediate section of the transition structure 200), an arbitrarily long axial section includes at least one first sub-region 1021 and at least one second sub-region 2001. In Embodiment 2, α and β open in the same direction. In some other embodiments, α and β may open in different directions. For example, α may open toward the trailing location 202 of the transition structure 200, while β may open toward the leading location 201 of the transition structure 200; and vice versa. It is to be noted that, in Embodiment 2, α and β refer to the acute angles formed between the planes defined by the end faces of the heterogeneous material zone 2 with respect to the axis of the catheter, rather than their obtuse complements.

In case of a constant inner diameter of the catheter, a greater difference between α and β leads to a greater difference between L1 and L2. In order to enable the catheter 100 to have smooth flexibility/stiffness transitioning to prevent the catheter 100 from kinking at any such transition site, in preferred embodiments of the present invention, the greater the stiffness difference between the first and second polymeric materials is, the smaller the angle(s) α and/or β is/are controlled to be, because the smaller the angle(s) α and/or β is/are, the milder the material variation at the transition site will be, and the more smoothly the mechanical properties of the catheter 100 there will vary, facilitating the passage of the catheter 100 through a tortuous blood vessel site with higher safety.

In Embodiment 2, the transition structure 200 has an axial section made up of only a second sub-region 2001 and not including any first sub-region 1011 (or 1021). As shown in Fig. 6, in some other embodiments, any axial section of the transition structure 200 includes at least one first sub-region 1011 (and/or first sub-region 1021) and at least one second sub-region 2001.

### Embodiment 3

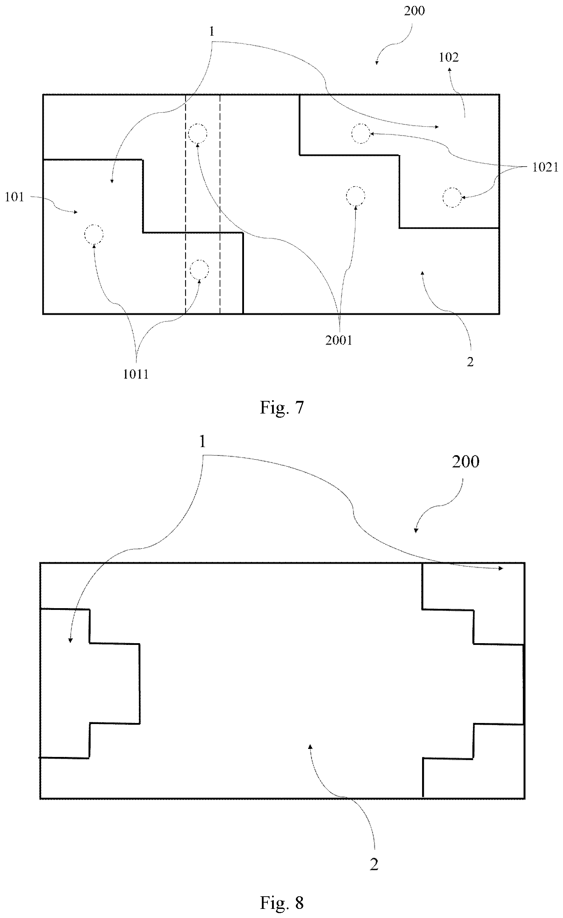

In Embodiment 3, there is provided a catheter 100 including at least one transition structure 200. As schematically shown in Fig. 7, each transition structure 200 in Embodiment 3 includes a homogeneous material zone 1 and a heterogeneous material zone 2. The homogeneous material zone 1 further includes a first polymeric region 101 and a second polymeric region 102. The first polymeric region 101 is made of a first polymeric material and the second polymeric region 102 of a second polymeric material. Stiffness of the first polymeric material is higher than stiffness of the second polymeric material. The first polymeric region 101 and the second polymeric region 102 are located on opposite sides of the heterogeneous material zone 2.

An axial projection of the heterogeneous material zone 2 of the transition structure 200 on a certain plane has stepped edges (in the form of rectangular polylines). In Embodiment 3, the edges at the opposite axial ends of the heterogeneous material zone 2 are both stepped. In other embodiments, only one of the edges of the heterogeneous material zone 2 in the transition structure 200 may be stepped.

In the transition structure 200 of the catheter 100 in Embodiment 3, any arbitrary axial section, such as that indicated by the dashed straight lines in Fig. 7, includes at least one second sub-region 2001. Moreover, in an axial portion of the transition structure 200 extending from an axial end provided by the first polymeric region 101 (located at a leading location of the transition structure 200) to an end position of the first polymeric region 101 (located in an intermediate section of the transition structure 200), an arbitrarily long axial section, such as that indicated by the dashed straight lines in the figure, includes at least one first sub-region 1011 and at least one second sub-region 2001. Likewise, in an axial portion of the transition structure 200 extending from an axial end provided by the second polymeric region 102 (located at a trailing location of the transition structure 200) to an end position of the second polymeric region 102 (located in the intermediate section of the transition structure 200), an arbitrarily long axial section includes at least one first sub-region 1021 and at least one second sub-region 2001.

Fig. 8 shows the transition structure 200 of Fig. 7 after it is cut along an axial line and unrolled flat. As can be seen from Fig. 8, the transition structure 200 of Fig. 7 is a horizontally symmetrical tubular structure. In some other embodiments, the transition structure 200 may be a tubular structure that is not horizontally symmetrical.

In some other embodiments, either or both of the edges of the heterogeneous material zone 2 at its opposite ends may be in the shape of polyline(s). Examples of the polyline(s) may include, but are not limited to, triangular, rectangular, square, sinusoidal, irregular and other polylines.

Other structural features of Embodiment 3 are similar to that of Embodiment 1 and, therefore, need not be described in further detail herein.

### Embodiment 4

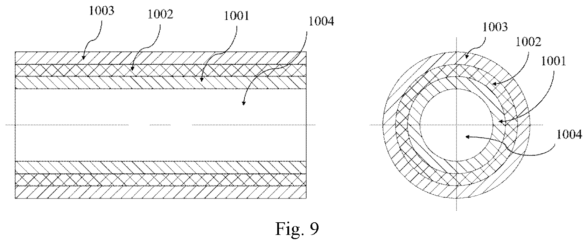

Fig. 9 shows axial and radial cross-sectional views of a catheter 100 according to Embodiment 4. As shown in Fig. 9, the catheter 100 includes an inner layer 1001, an intermediate reinforcing layer1002 and an outer layer1003 arranged radially from the inner side to the outer side. The reinforcing layer1002 is sleeved over the inner layer 1001, and the outer layer1003 is sleeved over the reinforcing layer1002. The inner layer 1001 defines a lumen 1004 of the catheter 100. The inner layer 1001 and the outer layer 1003 are both polymeric layers. In some other embodiments, the catheter 100 may include the outer layer 1003 and the inner layer 1001, and the reinforcing layer 1002 may be omitted. Alternatively, the catheter 100 may include only a single polymeric layer. Still alternatively, the catheter 100 may include only one polymeric layer and a reinforcing layer 1002.

The outer layer 1003 is a polymeric layer, which may be made of at least one selected from polyamide, polyolefin, poly(ether-block-amide) and polyurethane in embodiments of the present invention. In Embodiment 4, the outer layer 1003 is made of polyamide, poly(ether-block-amide), polyurethane and polyolefin. Specifically, the outer layer 1003 may be made of the above materials, which are joined together. In embodiments of the present invention, the reinforcing layer 1002 may be made of a metallic or polymeric material and functions to enhance strength, support performance, collapse resistance of the lumen, force transmission and torque control and transmission of the catheter 100. In Embodiment 4, the reinforcing layer 1002 is made of a metallic material. The inner layer 1001 is a polymeric layer, which may be made of at least one selected from polytrafluoroethylene, polyolefin, polyurethane and poly(ether-block-amide) in embodiments of the present invention. In Embodiment 4, the inner layer 1001 is made of materials including polytrafluoroethylene and polyolefin. Moreover, the inner layer 1001 includes a transition structure 200 as defined in any one of the first to third embodiments at a joint of the materials. In embodiments of the present invention, the catheter 100 may include at least one polymeric layer, which may include a transition structure 200 as defined in any one of the first to third embodiments. In some other embodiments, the catheter 100 may include a single polymeric layer, which may include a transition structure 200 as defined in any one of the first to third embodiments. Alternatively, the catheter 100 may include two polymeric layers: the inner layer 1001 and the outer layer 1003, either or both of which may include a transition structure 200 as defined in any one of the first to third embodiments. Still alternatively, the catheter 100 may include two or more polymeric layers, at least one of which may include a transition structure 200 as defined in any one of the first to third embodiments.

In Embodiment 4, the inner layer 1001 is made up of a first inner layer section located at the proximal end and a second inner layer section located at the distal end. The first inner layer section is made of a first polymeric material, and the second inner layer section of a second polymeric material. Stiffness of the first polymeric material is higher than stiffness of the second polymeric material. In embodiments of the present invention, the stiffness of the first polymeric material may range from 40 D to 70 D. The material may be, but is not limited to being, polytrafluoroethylene. In Embodiment 4, the stiffness of the first polymeric material is 60 D, and the material is polytrafluoroethylene. In some other embodiments, the stiffness of the first polymeric material may be 40 D, 45 D, 50 D, 54 D, 62 D, 65 D, 66 D, 68 D or 70 D. The stiffness of the second polymeric material may range from 30 A to 55 D. The material may be, but is not limited to being, any one of polyolefin, poly(ether-block-amide) and polyurethane, or a blend thereof. In Embodiment 4, the second polymeric material is polyolefin, and its stiffness is 35 D. In some other embodiments, the second polymeric material may be linear low-density polyethylene or polyolefin elastomer, poly(ether-block-amide) impregnated with lubricant, or polyurethane impregnated with lubricant. In some other embodiments, the stiffness of the second polymeric material may be 30 A, 40 A, 45 A, 60 A, 70 A, 80 A, 90 A, 30 D, 35 D, 38 D, 40 D, 50 D or 55 D. In embodiments of the present invention, the first inner layer section may have a length of 1000-1550 mm, and the second inner layer section may have a length of 50-600 mm. In Embodiment 4, the length of the first inner layer section is 1100 mm, and the length of the second inner layer section is 350 mm.

In other embodiments, the first inner layer section and/or the second inner layer section may be each made of a blend of several polymeric materials or a polymer mixed with an inorganic substance such as a radiopaque metal powder. In some other embodiments, the first inner layer section and/or the second inner layer section may be each made of polyolefin and poly(ether-block-amide) blended at a ratio of 1: 1. In some other embodiments, the first inner layer section and/or the second inner layer section may be each made of polyolefin and polyurethane blended at a ratio of 2: 1. In some other embodiments, the first inner layer section and/or the second inner layer section may be each made of poly(ether-block-amide) and polyurethane blended at a ratio of 1: 2. In some other embodiments, the first inner layer section and/or the second inner layer section may be each made of polyolefin, poly(ether-block-amide) and polyurethane blended at a ratio of 1: 1: 1. In some other embodiments, the first inner layer section and/or the second inner layer section may be each made of linear low-density polyethylene and polyolefin elastomer blended at a ratio of 1: 1. In some other embodiments, the first inner layer section and/or the second inner layer section may be each made of linear low-density polyethylene mixed with a tungsten powder, which can impart radiopaqueness to the body of the catheter 100. In some other embodiments, the first inner layer section and/or the second inner layer section may be each made of poly(ether-block-amide) with impregnated with lubricant. In some other embodiments, the first inner layer section and/or the second inner layer section may be each made of a 1: 1 blend of poly(ether-block-amide) and polyurethane impregnated with lubricant.

In one embodiment, the length of the first inner layer section may be 1000 mm, and the length of the second inner layer section may be 170 mm. In some other embodiments, the length of the first inner layer section may be 1550 mm, and the length of the second inner layer section may be 50 mm. In some other embodiments, the length of the first inner layer section may be 1150 mm, and the length of the second inner layer section may be 600 mm. In some other embodiments, the length of the first inner layer section may be 200 mm, and the length of the second inner layer section may be 1200 mm.

### Embodiment 5

In Embodiment 5, there is provided a method of making a transition structure 200 as defined in any one of the first to fourth embodiments. The method provided in the fifth embodiment includes cutting first and second polymeric tubes at their ends so that the first polymeric tube and/or the second polymeric tube each have/has a axially extent at the end, which is smaller than a complete circumference. The first polymeric tube is made of a first polymeric material and the second polymeric tube of a second polymeric material. Stiffness of the first polymeric material is higher than stiffness of the second polymeric material. The first and second polymeric tubes are at least partially piled and joined together at their ends, and the transition structure 200 is then obtained by subjecting them to heat shrinkage.

In the method provided in the fifth embodiment, the transition structure 200 is not separately fabricated and then assembled in a catheter 100. Rather, it is indeed formed during the fabrication of the catheter 100 (or of a polymeric layer thereof). For example, the first polymeric tube may be actually a precursor of a proximal section of an inner layer 1001 in the catheter, and the second polymeric tube may be indeed a precursor of a distal section of the inner layer 1001 in the catheter. Therefore, the inner layer 1001 of the catheter 100 is eventually obtained from the above process performed on the first and second polymeric tubes in the method, and the transition structure 200 is located at the inner layer 1001 where the materials interface each other. Alternatively, the catheter may be a multi-layered structure, in this case, polymeric precursor tubes for any polymeric layer in the structure may be cut at ends, assembled in the structure and subjected to heat shrinkage. In this way, the complete catheter 100 may be eventually obtained, which contains the transition structure 200 as a material transition portion of the polymeric layer.

In Embodiment 5, the heat shrinkage process is performed at a temperature lying between the melting points of the first and second polymeric materials. As a result of the heat shrinkage process, a heterogeneous material zone 2 in the transition structure 200 is formed by both the first and second polymeric materials that are overlapped and joined to each other. The heterogeneous material zone 2 is formed as a composite structure. In some other embodiments, the shrinkage process is performed at a temperature higher than both the melting points of the first and second polymeric materials. In these cases, as a result of the heat shrinkage process, the heterogeneous material zone 2 is formed of the first and second polymeric materials, which are blended and fused together. That is, the heterogeneous material zone 2 is formed of a blend of the two materials.

In some other embodiments, before the first and second polymeric tubes are cut at their ends, or after the first and second polymeric tubes are cut at their ends and before the first and second polymeric tubes are piled and joined together, the first polymeric tube and/or the second polymeric tube may be stretched to have a reduced wall thickness at said end. As the thickness at the stretched ends is smaller than the wall thickness at remaining positions of the tube, after the tubes are joined together at the ends, the catheter 100 can have a relatively small wall thickness at the joint. This enables the catheter 100 to have an overall uniform size across its entire length without any abrupt change in its outer diameter. As such, the catheter 100 can have even smoother transitioning of overall mechanical properties and hence additionally enhanced delivery performance. For some particular materials, properly stretching the tubes can increase their strength. As a result, the joint will exhibit higher strength, preventing easy separation of the tubes at the joint when they are pulled.

Although the present invention has been disclosed hereinabove, it is not limited to the above disclosure. Those skilled in the art can make various changes and modifications to the invention without departing from the spirit and scope thereof. Accordingly, it is intended that any and all such changes and modifications also fall within the scope of the present invention as defined by the appended claims and equivalents thereof.

## Claims

1. A catheter, comprising at least one polymeric layer, wherein the polymeric layer comprises at least one transition structure that is a tubular structure, wherein the transition structure comprises a homogeneous material zone and a heterogeneous material zone, wherein: any continuous closed region of the homogeneous material zone is referred to as a first sub-region; any continuous closed region of the heterogeneous material zone as a second sub-region; and any section of the transition structure along an axis comprises at least one second sub-region, and wherein a leading section and/or a trailing section of the transition structure comprise(s) at least one first sub-region and at least one second sub-region.

2. The catheter of claim 1, wherein any axial section of the transition structure comprises at least one first sub-region and at least one second sub-region.

3. The catheter of claim 1, wherein axially from the leading location and/or the trailing location of the transition structure at respective end(s) to an intermediate section of the transition structure, an outer surface area ratio of the homogeneous material zone to the heterogeneous material zone gradually decreases.

4. The catheter of claim 2, wherein from a first end to a second end of the transition structure, an outer surface area ratio of the homogeneous material zone to the heterogeneous material zone gradually decreases to a minimum value and then gradually increases.

5. The catheter of claim 1, wherein at least one end face of the heterogeneous material zone defines a plane which is inclined with respect to an axis of the catheter at an angle.

6. The catheter of claim 5, wherein the plane is inclined with respect to the axis of the catheter at an angle ranging from 5° to 60°.

7. The catheter of claim 1, wherein at least one edge of the heterogeneous material zone has a polylineal structure.

8. The catheter of claim 1, wherein the heterogeneous material zone has a constant axial length in a range of 1-15 mm.

9. The catheter of claim 1, wherein the heterogeneous material zone has a varying axial length with a maximum value of 3-20 mm and a minimum value of 0.5-15 mm.

10. The catheter of any one of claims 1 to 9, wherein the homogeneous material zone comprises a first polymeric region and a second polymeric region situated on opposite sides of the heterogeneous material zone, wherein the first polymeric region is made of a first polymeric material, and the second polymeric region is made of a second polymeric material, and wherein a stiffness of the first polymeric material is higher than a stiffness of the second polymeric material.

11. The catheter of claim 10, wherein a stiffness of the heterogeneous material zone is lower than the stiffness of the first polymeric region and is higher than the stiffness of the second polymeric region.

12. The catheter of claim 10, wherein at least one end face of the heterogeneous material zone defines a plane which is inclined with respect to an axis of the catheter at an angle, and wherein a greater stiffness difference between the first and second polymeric materials is, a smaller angle is formed.

13. The catheter of claim 10, wherein the heterogeneous material zone is made of the first and second polymeric materials, which are blended and fused.

14. The catheter of claim 10, wherein the heterogeneous material zone is made of the first and second polymeric materials, which are piled and joined.

15. The catheter of claim 10, wherein the first polymeric material is any one of polytrafluoroethylene, polyolefin, polyurethane, poly(ether-block-amide) and polyamide, and wherein the second polymeric material is any one of polytrafluoroethylene, polyolefin, polyurethane, poly(ether-block-amide) and polyamide.

16. The catheter of claim 1, further comprising a reinforcing layer.

17. The catheter of claim 1, wherein the catheter is a triple-layered structure comprising an inner layer, an intermediate reinforcing layer and an outer layer, wherein the inner and outer layers are polymeric layers, and wherein the inner layer comprises at least one transition structure and/or the outer layer comprises at least one transition structure.

18. The catheter of claim 17, wherein the inner layer comprises at least one transition structure.

19. The catheter of claim 18, wherein a distal section of the inner layer comprises the at least one transition structure.

20. The catheter of claim 17, wherein the outer layer comprises at least one transition structure.

21. A method of making a transition structure of a catheter, comprising: cutting an end of a first polymeric tube and an end of a second polymeric tube so that an end portion of the first polymeric tube and/or an end portion of the second polymeric tube have/has a circumferential extent which is smaller than a complete circumference, wherein the first polymeric tube is made of a first polymeric material, wherein the second polymeric tube is made of a second polymeric material, wherein a stiffness of the first polymeric material is higher than a stiffness of the second polymeric material; and wherein the end of the first polymeric tube and the end of the second polymeric tubes are at least partially piled and joined and then subjected to a heat shrinkage process.

22. The method of claim 21, wherein the heat shrinkage process is carried out at a temperature higher than melting points of the first and second polymeric materials.

23. The method of claim 21, wherein the heat shrinkage process is carried out at a temperature lying between a melting point of the first polymeric material and a melting point of the second polymeric material.

24. The method of claim 21, further comprising, prior to the piling and joining, stretching the end of the first polymeric tube and/or the end of the second polymeric tube, so that for each tube, a wall thickness at a stretched end is smaller than a wall thickness at remaining positions of the tube.
